# EUROPEAN PATENT APPLICATION

(11) **EP 3 536 207 A1**
(43) Date of publication of application: **11.09.2019**
(21) Application number: 18160820.9
(22) Date of filing: 08.03.2018
(51) Int. Cl.: A47K 10/46, B65F 1/14

(54) **DEVICE FOR PERSONAL HYGIENE, PROCESS AND USES THEREOF**

(30) Priority: 05.03.2018 PT 2018110608
(71) Applicant: Inegi - Instituto de Ciência e Inovação em Engenharia Mecânica e Engenharia Industrial, 4200-465 Porto (PT)
(72) Inventor: Marques da Silva, João Paulo, 4470-808 Vila Nova da Telha - Maia (PT); Lourenço Reis, Ana Rosanete, 4050-612 Porto (PT); Bastos Fula, Diogo Emanuel, 4250-076 Paranhos; Porto (PT); Lopes de Brito Seara Cardoso, Ricardo, 4100-442 Porto (PT); Salgado Cebola, Peter Edward, 4410-283 Canelas (PT)
(74) Representative: Patentree

(57) **Abstract**

Device for personal hygiene comprising a dispenser of personal hygiene tissues, a motorised antibacterial fluid dispenser and a motorised collector of used tissues; wherein the motorized collector comprises a waste container for collecting and compacting used tissues; wherein the fluid dispenser is arranged for dispensing antibacterial fluid to the used tissues at the waste container. The motorised collector preferably comprises two motorised compactor rolls, wherein the rolls have a shape of horizontal cylindrical segments and are arranged to rotate in opposite directions for gathering and compacting the used tissues into the waste container. The motorised collector preferably comprises two scrapers for scraping the used tissues from the compactor rolls into the waste container. The motorised antibacterial fluid dispenser is preferably arranged for dispensing the antibacterial fluid to the motorised collector of used tissues for lubricating the motorised collector.

## Description

### FIELD OF INVENTION

The present disclosure relates to a personal hygiene tissue dispenser, a fluid dispensing device having a bacteriostatic action and a motorized collector unit, particularly designed to work in spaces such as public bathrooms.

It is disclosed a personal, bacteriostatic dispenser and a waste container capable of compacting used tissues, maintaining them hidden from site and restricting bad smells and bacteria by means of bacteriostatic agent.

### BACKGROUND

A main goal of the present disclosure, relates to the lack of personal hygiene solutions near the urinal in gent's bathrooms. This problem has been previously tackled in several manners, but never fully solved. The problem divides itself in several challenges.

Following urination, men do not have a personal hygiene solution in order to eliminate the vestiges of remaining urine. This can cause embarrassing spotting or staining in the event that urine is inadvertently splashed onto one's clothing or transpose onto one's underwear. Furthermore, individuals with urinary complications, may experience a residual urinary stream after urinating that cannot be solved by ordinary methods.

Men do not have access to any kind of toilet paper or tissue near the urinal, for what men usually do not clean themselves after using it. Although, during our survey study, we found that many men do not use the urinal, instead they resort to the toilet in order to have access to toilet paper. At the same time, we found that some religions encourage this kind of behaviour, to the point of, in some cases, providing good practice rules for men.

The space surrounding a urinal is almost always very small, not allowing a lot of free room so a tissue/paper dispenser and discarder may coexist in the same place. Without proper building regulation, this is expected to continue to be a problem.

Although many men use toilet paper for cleaning themselves up, the material from which is made is neither comfortable, neither resistant enough for the purpose. Different kinds of tissue papers are available for men to buy online, with which they can clean themselves, but this require men to travel everywhere with those products. Although the market for these products has been growing, we believe that this solution is not ideal, as we consider that an In Situ complete solution is more favourable for the end user.

In case a paper dispenser is provided for the purpose, the gathering of used paper with reminiscence of urine is considered a danger to public health due to bacteria, and also a place for bad odors to occur. Proper disposal of the resultant paper is required in order to reach a complete solution.

Currently, there is one commercially available solution that seems to tackle this problem. The "Dry Sec" product from the company ISSA, having corresponding patent document WO2010079372 (A1), and having a mechanism that holds the plastic bag inside the product. The Dry Sec uses regular hand whipping paper; it seems to be too wide to coexist between urinals (in most cases); its existence blocks the possibility of using a bacteriostatic discharger in the urinal as it consumes the same space; the discard bin is too small to withstand all the tissues that can be discarded; it does not cover the odours of urine from the discarded papers; in case that the bin gets full of papers, its normal behaviour that people keep stacking up papers until it collapses the last one being a serious issue with this current design.

Our surveys revealed that it is also not visually pleasant to people to be able to see the discarded paper. For this reason, the Dry Sec seems to have still a long way to go in order to become a good solution for the current problems.

There are more traditional ways to address the problem using household paper dispensers, however, users will discard papers directly in the urinal, creating clogs. This can be prevented by suppling a paper bin so users can discard the used paper tissues, however this will have the same problems as stated in the previous solution "Dry Sec".

The system presented in EP1508647 is another solution to the same problem. Although the paper dispenser remains the same as the previous one, the discard unit is mounted in the wall, using a sewer line to discard the resultant paper. This idea is described in the EP1508647 A1 patent document; nevertheless, we could not find much more information about this solution beyond the patent, which may be indicative that the project never became fulfilled.

This is also true on the product in document US3549226, with the difference of the discarded material, which in this case are cotton disks instead of paper tissues. It shares the same problem as the previous solution, for which additional information is non-existing.

These facts are disclosed in order to illustrate the technical problem addressed by the present disclosure.

### GENERAL DESCRIPTION

The present invention relates to an apparatus that comprises a personal hygiene tissue dispenser, bacteriostatic dispenser and a waste container capable of compacting used tissues, maintaining them hidden from site and restricting bad smells and bacteria by means of bacteriostatic agent.

The top compartment is used as a paper dispenser, comprising a slot from which clean tissues can be drawn, a compartment to sustain the paper tissues in place, a spring that maintains pressure in the back of the tissues stack making possible to remove every single tissue. This part of the system is within the upper cover that pivots in an internal hinge, making it possible to recharge the paper stack and the bacteriostatic as well as access the control buttons and the printed-circuit-board, PCB.

Tissues, paper tissues and papers are used interchangeably in the context of the present disclosure.

In the back of the upper part resides a bacteriostatic charge, an engine responsible for turning a cam mechanism, the cam that is used to pressure activate the bacteriostatic charge nozzle and the control PCB for the complete system.

The waste receptacle is organized in three main elements: a discard receptacle; a compacter system and a discard bin.

The first being a receptacle of small dimensions, appropriated so one can discard a used tissue without having to wait for the compacting unit to actuate. It also gives room so the compacting system can accommodate several tissues before actuating, this not only can prevent jams, it also increases battery lifetime and sustains the infrared sensors that actuate the roller system.

The roll system is activated once the sensors detect the presence of tissues (not exclusively) in the first receptacle, turning the rolls in opposite directions, gathering and compacting, the used tissues in the discard bin, which is positioned directly beneath the roll system. The bin is comprised of a series of sheet metal parts that hold in place a garbage bag that can be changed periodically in an orderly and non-hazardous way through the front bin door.

It is disclosed a device for personal hygiene comprising a dispenser of personal hygiene tissues, a motorised antibacterial fluid dispenser and a motorised collector of used tissues;
wherein the motorized collector comprises a waste container for collecting and compacting used tissues,
wherein the fluid dispenser is arranged for dispensing antibacterial fluid to the used tissues at the waste container.

An antibacterial fluid is a liquid that is active against bacteria. An antibacterial fluid can be a bacteriostatic fluid or bactericide fluid.

An embodiment comprises the motorised collector, comprises two motorised compactor rolls, wherein the rolls have a shape of horizontal cylindrical segments and are arranged to rotate in opposite directions for gathering and compacting the used tissues into the waste container.

Horizontal cylindrical segment is a solid cut from a horizontal cylinder by a surface oriented parallel to the cylinder's axis of symmetry (i.e., a portion of a horizontal cylindrical tank which is partially filled with fluid).

In an embodiment, the motorised collector comprises two scrapers for scraping the used tissues from the compactor rolls into the waste container.

In an embodiment, the motorised antibacterial fluid dispenser is arranged for dispensing antibacterial fluid to the motorised collector of used tissues for lubricating the motorised collector.

In an embodiment, the motorised antibacterial fluid dispenser comprises an antibacterial fluid charge having a nozzle and a motorised cam for pressure activating the antibacterial fluid charge nozzle.

In an embodiment, the motorised collector of used tissues comprises a discard receptacle for receiving the used tissues and a sensor for detecting the presence of used tissue or tissues in the discard receptacle.

In an embodiment, an electronic control circuit configured for activating the collecting and compacting of used tissues by the motorized collector that actuates when the sensor detects the presence of used tissue or tissues in the discard receptacle.

In an embodiment, an electronic control circuit configured for activating the collecting and compacting of used tissues by the motorized collector that actuates when the sensor detects the presence of a plurality of used tissues in the discard receptacle.

In an embodiment, the antibacterial fluid is a bacteriostatic fluid.

In an embodiment, the tissue dispenser is in a top compartment of the device.

In an embodiment, the fluid dispenser is in a top compartment of the device.

In an embodiment, the motorised collector of used tissues is in a bottom compartment of the device.

In an embodiment, the tissue dispenser comprises a hinged cover for covering the tissue dispenser and for opening for reloading the tissue dispenser with tissues.

In an embodiment, the tissue dispenser comprises a spring for maintaining pressure at the end of a tissue stack for dispensing individual tissues.

In an embodiment, the waste container comprises a discard bin for collecting the used tissues and for holding a garbage bag suitable to be changed.

In an embodiment, the discard bin is positioned directly beneath the compactor rolls.

### BRIEF DESCRIPTION OF THE DRAWINGS

The following figures provide preferred embodiments for illustrating the description and should not be seen as limiting the scope of invention.
**Fig. 1****:** illustrates an embodiment of the disclosed device.
**Fig. 2****:** illustrates an embodiment of an upper section of the disclosed device when closed.
**Fig. 3****:** illustrates a upper section of the disclosed device with opened cover.
**Fig. 4****:** illustrates an embodiment of the disclosed device upper cover paper and/or tissue dispenser unit.
**Fig. 5****:** illustrates an embodiment of the disclosed device upper section bacteriostatic discharger mechanism, the bacteriostatic pump system.
**Fig. 6****:** illustrates an embodiment of the upper section of the disclosed device: paper and/or tissue discard and compaction system.
**Fig. 7****:** illustrates an embodiment of the upper section of the disclosed device battery system and control buttons.
**Fig. 8****:** Illustrates an embodiment of the lower section of disclosed device a garbage bin system and door.

### DETAILED DESCRIPTION

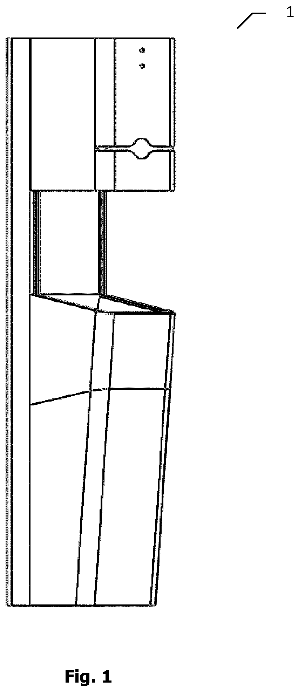

In an embodiment, the **figure 1** illustrates a disclosed device **1** that comprises a personal hygiene tissue dispenser, bacteriostatic dispenser and a waste container.

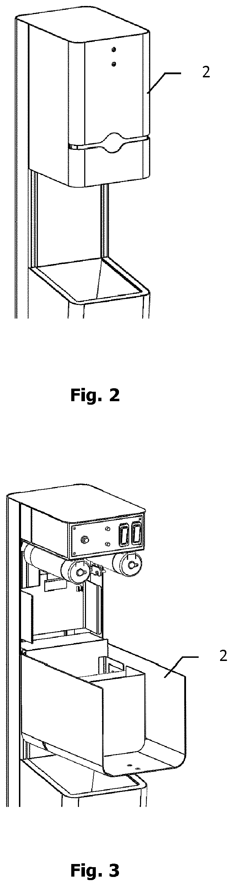

In an embodiment, the **figure 2** illustrates an upper section of the disclosed device with the cover **2** closed.

In an embodiment, the **figure 3** illustrates a upper section of the disclosed device with opened cover **2.**

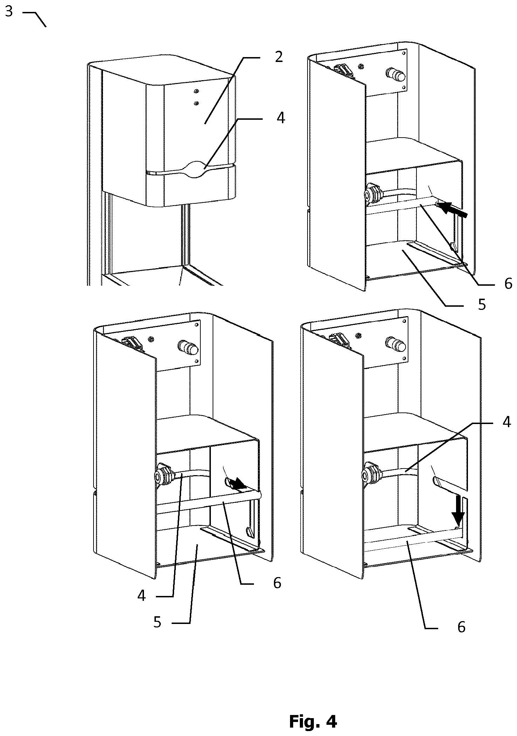

In an embodiment, the **figure 4** illustrates the top compartment that is used as a paper dispenser, comprising a slot from which clean tissues can be drawn, a compartment to sustain the paper tissues in place, a spring that maintains pressure in the back of the tissues, stack making possible to remove every single tissue. This part of the system is within the upper cover that pivots in an internal hinge, making it possible to recharge the paper stack.

The dispenser unit **3** is comprised of three main elements: the outside sheet metal cover **2** which follows the overall design and allows the paper output through the dispensing aperture **4;** the container **5** that dispenses tissues of the type which are pre-folded and stacked in interleaved fashion so that when one tissue is extracted from the dispenser aperture **4,** a portion of the following tissue is partially withdrawn and may be readily grasped. In order that each succeeding tissue may be readily extracted, a spring-pressed follower tube **6** is employed so it constantly places small amounts of pressure urging the stack of tissue toward the dispensing aperture **4.**

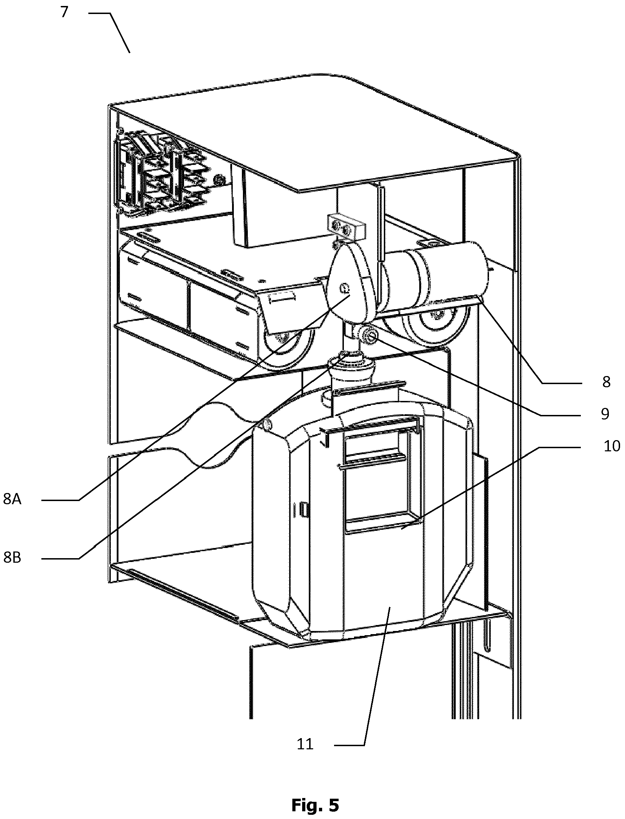

In an embodiment, the **figure 5** illustrates a bacteriostatic fluid dispensing device **7** operating continuously so to prevent return of air and comprising a pump **10** mounted on a container **11** comprising a fluid, a nozzle **9** and a cam mechanism **8A** with a pusher **8B** for operating the pump **10.** At the system start-up, the motor **8** activates the pump **10** a set number of times and places the cam mechanism **8A** in a known reference position.

After a set period of time has been elapsed (chosen by the user), the system rotates to activate the cam mechanism **8A** and stops in the reference position. The pusher **8B** actuates a nozzle **9** which activates the pump **10,** thus dispensing the substance that has a bacteriostatic effect. The nozzle **9** as narrow low-volume discharge channel between the pump **10** and the dispensing opening, and the bacteriostatic active substance is provided only in the pusher **8B** on or adjacent to the nozzle **9** so that the effectiveness of the bacteriostatic active substance is maximized as a result of the small void volume in the pusher **8B.**

The bacteriostatic discharge can be delimited by a switch in order to exhaust the container between two different periods of time. (15 days or one month). A set of different diameter tubes are used to steer the bacteriostatic agent to the adjacent urinals and the integrated bin with used tissues, thus reducing bad odours and avoiding the proliferation of bacteria inside the bin.

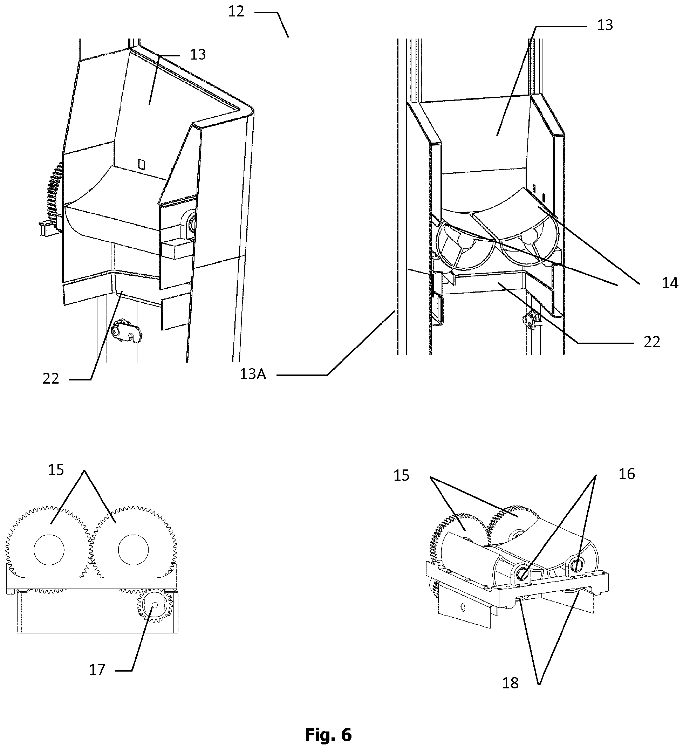

In an embodiment, the **figure 6** illustrates a tissue discard system **12** that includes a reservoir **22** and a pre-reservoir **13,** where tissues are thrown to, made so that the papers inside the container **13A** are invisible to the user and where the paper detection module is mounted. This pre-reservoir **13** is made of two special shaped cylinders **14** that revolve along their axle. They are actuated by an electric motor **17** connected to one small gear **17A,** and the gear ratio is calculated so that there is sufficient torque to compact the papers at all circumstances.

The two cylinders **14** are connected by two gears **15,** solidary with each respective cylinder **14,** with the same number of teeth (1:1 ratio) so that the cylinder synchronization is guaranteed on every occasion. Thanks to their geometry, the cylinders **14** are able to compact the paper inside the reservoir **22,** allowing a bigger paper capacity, consequently reducing time between bag disposals.

Maintenance-free polymeric bearings **16** guarantee a smooth movement and a reduced play so that gear interference and any mechanical blockage is avoided. Under the cylinders **14,** two scrappers **18** insure a correct cleaning of the cylinder's surface, and prevent papers from going back up to the pre-reservoir **13.**

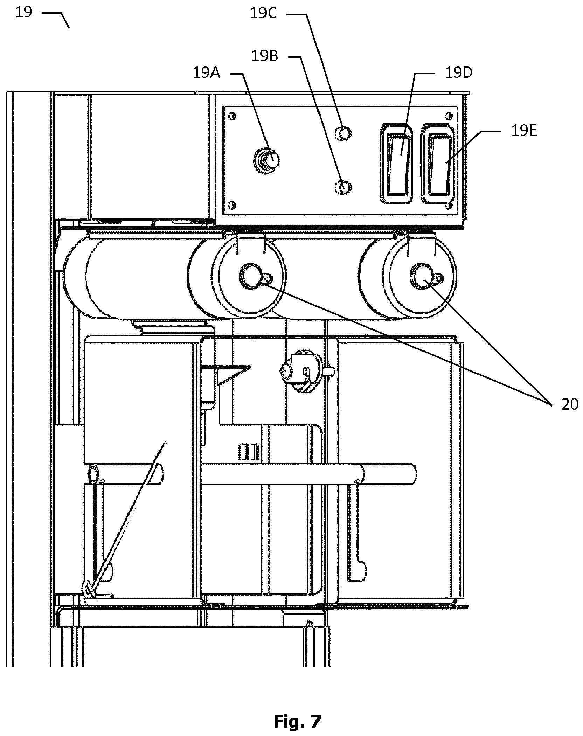

In an embodiment, the **figure 7** illustrates a control system **19** that comprises three main subsystems: the infrared (IR) detection system, the cylinder control system and the bacteriostatic control system. The infrared detection system consists in IR LEDs and IR receptors, which detects the presence of a tissue in the discard receptacle.

This is achieved by emitting a modulated signal from the LED to the receptor that will be interrupted by the tissue, thus informing the system. In case that the signal is continuously interrupted, instead of informing the main control loop that a paper is being detected, a system failure signal is sent; The cylinder system **14** actuates when a signal generated from the infrared detection system is received.

At the system startup, the motor **17** rotates the cylinder **14** to a known reference position. In normal operating conditions, the motor **17** activates the cylinders **14,** pushing tissues to the discard bin **21,** stopping in the reference position. In case of inability to rotate the cylinders **14** (that is, the motor draws electric current over a defined threshold), the motor **17** will reverse its rotation to the reference position in order to try again. This procedure always repeats when the system is under these conditions, up to a defined number of tries. If the number of attempts is superior, the motor **17** ceases its functioning and signals a system failure to the main control loop.

The bacteriostatic system controls the actuation of the bacteriostatic motor 8 for pump system **10.** At the system startup, the motor activates the pump a set number of times and places the cam mechanism in a known reference position. After a set period of time has been elapsed (chosen by the user), the system rotates to activate the cam mechanism and stops in the reference position. Both motor control system possesses switches to provide a reference position. In case of failure of these switches, a system failure signal is also sent to the main control loop.

An user interface exists in the upper section of the disclosed device for interaction with the system, composed by a push button **19A** to reset the system after a critical failure or after restocking the bacteriostatic fluid, two switches **19D, 19E** (one for controlling the power supply **19D** and the other to choose between two rates of bacteriostatic usage **19E**), a green LED **19B** to provide feedback on the system's operation to the user (to inform if the system is on) and a red LED **18C** to provide feedback on the bacteriostatic charge (different patterns of blinking depending on the quantity available) and to inform if a system failure has occurred (permanently turned on). The control system of the disclosed device is powered by the batteries **20.**

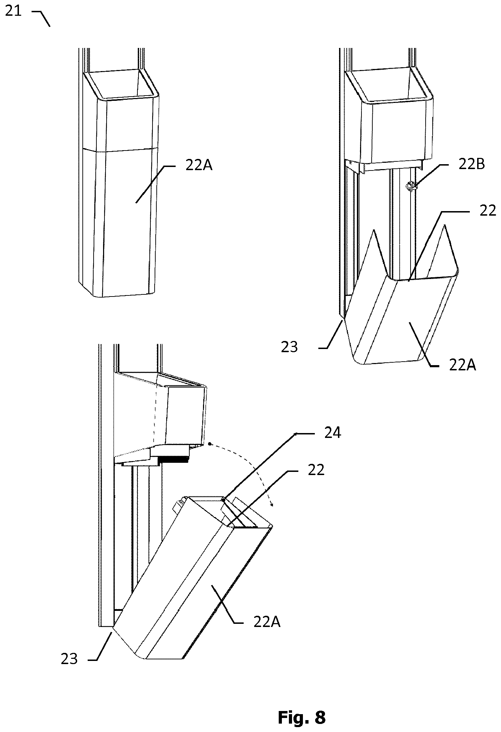

In an embodiment, the **figure 8** illustrates a garbage disposal system **21** that comprises a trash container cover **22A** with a key releasing mechanism **22B** that allows the container **22** to rotate in a hinge **23,** therefore, a user is able to easily remove the filled trash bag to be disposed and allows fitting of new clean bags.

The trash container **22** of the present invention enables removal of the trash bag without requiring lifting of the filled trash bag to the top of the trash container **22.** In this manner, the filled trash bag can be dragged out of the garbage bin for later discard. Inside the cover, a ring **24** is used to accommodate the top of the garbage bag using a locking ring, this allows easy placement and removal of the trash bags.

The disclosure should not be seen in any way restricted to the embodiments described and a person with ordinary skill in the art will foresee many possibilities to modifications thereof. The above described embodiments are combinable. The following claims further set out particular embodiments of the disclosure.

## Claims

1. Device for personal hygiene comprising a dispenser of personal hygiene tissues, a motorised antibacterial fluid dispenser and a motorised collector of used tissues;
wherein the motorized collector comprises a waste container for collecting and compacting used tissues;
wherein the fluid dispenser is arranged for dispensing antibacterial fluid to the used tissues at the waste container.

2. Device according to the previous claim wherein the motorised collector comprises two motorised compactor rolls, wherein the rolls have a shape of horizontal cylindrical segments and are arranged to rotate in opposite directions for gathering and compacting the used tissues into the waste container.

3. Device according to claim 2 wherein the motorised collector comprises two scrapers for scraping the used tissues from the compactor rolls into the waste container.

4. Device according to any of the previous claims wherein the motorised antibacterial fluid dispenser is arranged for dispensing the antibacterial fluid to the motorised collector of used tissues for lubricating the motorised collector.

5. Device according to any of the previous claims wherein the motorised antibacterial fluid dispenser comprises an antibacterial fluid charge having a nozzle and a motorised cam for pressure activating the antibacterial fluid charge nozzle.

6. Device according to any of the previous claims wherein the motorised collector of used tissues comprises a discard receptacle for receiving the used tissues and a sensor for detecting the presence of used tissue or tissues in the discard receptacle.

7. Device according to the previous claim comprising an electronic control circuit configured for activating the collecting and compacting of used tissues by the motorized collector when the sensor detects the presence of used tissue or tissues in the discard receptacle.

8. Device according to the previous claim comprising an electronic control circuit configured for activating the collecting and compacting of used tissues by the motorized collector when the sensor detects the presence of a plurality of used tissues in the discard receptacle.

9. Device according to any of the previous claims wherein the antibacterial fluid is a bacteriostatic fluid.

10. Device according to any of the previous claims wherein the tissue dispenser is in a top compartment of the device.

11. Device according to any of the previous claims wherein the fluid dispenser is in a top compartment of the device.

12. Device according to any of the previous claims wherein motorised collector of used tissues is in a bottom compartment of the device.

13. Device according to any of the previous claims wherein the tissue dispenser comprises a hinged cover for covering the tissue dispenser and for opening for reloading the tissue dispenser with tissues.

14. Device according to any of the previous claims wherein the tissue dispenser comprises a spring for maintaining pressure at the end of a tissue stack for dispensing individual tissues.

15. Device according to any of the previous claims wherein the waste container comprises a discard bin for collecting the used tissues and for holding a garbage bag suitable to be changed, in particular wherein the discard bin is positioned directly beneath the compactor rolls.
